Europäisches Patentamt

European Patent Office

Office européen des brevets

(11) Veröffentlichungsnummer: **0 513 738 A2**

# EUROPÄISCHE PATENTANMELDUNG

(21) Anmeldenummer: **92107997.6**

(51) Int. Cl.5: **C12N 5/00**, C12N 5/06

(22) Anmeldetag: **12.05.92**

(30) Priorität: **14.05.91 DE 4115722**

(43) Veröffentlichungstag der Anmeldung:
**19.11.92 Patentblatt 92/47**

(84) Benannte Vertragsstaaten:
**AT BE CH DE DK ES FR GB GR IT LI LU NL PT SE**

(71) Anmelder: **BOEHRINGER MANNHEIM GMBH
Sandhofer Strasse 112-132
W-6800 Mannheim-Waldhof(DE)**

(72) Erfinder: **Koch, Stefan, Dr.
Lagoner Strasse 18
W-8122 Penzberg(DE)**
Erfinder: **Behrendt, Ulrich, Dr.**
Heimgartenstrasse 5
**W-8177 Bichl(DE)**
Erfinder: **Franze, Reinhard, Dr.
Am Schwadergraben 11
W-8122 Penzberg(DE)**
Erfinder: **Lorenz, Thomas, Dr.
Grube 10 A
W-8122 Penzberg(DE)**
Erfinder: **Szperalski, Berthold, Dr.
Birkenstrasse 133
W-8122 Penzberg(DE)**

(74) Vertreter: **Böhm, Brigitte, Dipl.-Chem., Dr. et al
Kopernikusstrasse 9, Postfach 86 08 20
W-8000 München 86(DE)**

(54) **Serumfreies Medium zur Kultivierung von Säugerzellen.**

(57) Ein serumfreies Medium zur Kultivierung von Säugerzellen ohne Proteinmaterial tierischen Ursprungs, enthält neben üblichen Inhaltsstoffen anstelle von tierischem Insulin und Transferrin rekombinantes Insulin aus Prokaryonten und eine wasserlösliche Eisenverbindung. Ein derartiges Medium wird erfindungsgemäß zur Kultivierung von Säugerzellen, insbesondere CHO-Zellen verwendet.

Fig.1

SERUMFREIE KULTIVIERUNG VON CHO-ZELLEN IM 10L-FERMENTER: LEBENDZELLDICHTE

EP 0 513 738 A2

Die Erfindung betrifft ein serumfreies Medium zur Kultivierung von Säugerzellen ohne Proteinmaterial tierischen Ursprungs, sowie ein Verfahren zur Kultivierung von Säugerzellen, insbesondere CHO-Zellen, unter Verwendung eines derartigen Mediums und schließlich ein Verfahren zur gentechnologischen Herstellung von Erythropoietin, bei dem entsprechend transformierte CHO-Zellen in dem erfindungsgemäßen Kulturmedium wachsen.

Für das Wachstum tierischer Zellen in Kultur ist als Mediumzusatz im allgemeinen Serum, meist fötales Kälberserum, Voraussetzung. Dabei wird zu einem bestimmte Grundkomponenten, wie z.B. anorganische Salze, Aminosäuren, Monosaccharide, Vitamine etc., enthaltenden Grundmedium ein Zusatz einer bestimmten Menge von Serum beigemischt, wodurch den Zellen möglichst günstige Bedingungen für das Wachstum in der Zellkultur geboten werden.

Nun wurde bereits versucht, das Serum durch Ersatzstoffe in bekannten serumfreien Medien auszutauschen. So ist z.B. aus der DE-OS 37 33 453 ein serumfreies Medium zur Kultivierung von Hybridom- und Myelomzellen bekannt, das in einem synthetischen Kultivierungsmedium neben anderen, bekannten Zusatzstoffen eine wasserlösliche Eisenverbindung enthält. In der Beschreibung wird hierzu ausgeführt, daß diese wasserlösliche Eisenverbindung ein Ersatzstoff für das im Serum enthaltene Transferrin darstellt. Für die Kultivierung von Hybridom- und Myelomzellen sind gemäß dieser Offenlegungsschrift die dort genannten Medien verwendbar.

In Serum sind jedoch weitere Stoffe enthalten, die das Wachstum von Säugerzellen beeinflussen können. Neben dem Transferrin sind hier insbesondere noch Insulin und Albumin zu nennen. Für die Kultivierung von Säugerzellen ganz allgemein sollte daher bei Verwendung eines serumfreien Mediums möglichst darauf geachtet werden, den Bedingungen, wie sie unter Zugabe von Serum vorliegen, möglichst nahe zu kommen. Für die Herstellung von Therapeutika sind jedoch die Einsatzstoffe kritisch. Hierbei werfen die Zulassungsbehörden bei Zellkulturprodukten unter anderem besonderes Augenmerk auf Proteine als Mediumzusatzstoffe, die tierischen oder humanen Ursprungs sind, weil über diese Komponenten z.B. auch pathogene Viren in die Zellkulturmedien und eventuell ins Endprodukt gelangen können.

Aufgabe der Erfindung war es daher, ein serumfreies Kultivierungsmedium für Säugerzellen ganz allgemeiner Art zu schaffen, das die Kultivierungsbedingungen unter Verwendung eines Mediums mit Serum sehr nahekommt, jedoch kein Proteinmaterial tierischen Ursprungs enthält, was eine Gefahr von viraler Kontamination darstellen könnte.

Gelöst wird diese Aufgabe durch ein serumfreies Medium zur Kultivierung von Säugerzellen ohne Proteinmaterial tierischen Ursprungs, enthaltend neben üblichen Inhaltsstoffen anstelle von tierischem Insulin und Transferrin rekombinantes Insulin aus Prokaryonten und eine wasserlösliche Eisenverbindung.

Durch die Beibehaltung des Insulin im erfindungsgemäßen Medium sind die Kulturbedingungen deutlich ähnlicher zu Kultivierungsbedingungen unter Verwendung von Serum als dies bei bekannten serumfreien Kultivierungsmedien der Fall ist. Durch Einsatz von rekombinantem Insulin aus Prokaryonten im erfindungsgemäßen Medium wird außerdem die Gefahr viraler Kontaminationen vermieden.

Übliche Inhaltsstoffe von Kultivierungsmedien sind dem Fachmann bekannt und sind beispielsweise die Inhaltsstoffe, die in den Medien Dulbecco's modified Eagle Medium (Virology 8 (1959) 396) oder im Medium F12 (Ham's F12 Medium, Proc.Natl. Acad.Sci. USA 53 (1965) 288) enthalten sind. Dabei handelt es sich u.a. um Aminosäuren, Vitamine sowie andere Komponenten wie Glucose, Natriumpyruvat, bestimmte Fettsäuren und anorganische Salze.

In einer bevorzugten Ausführungsform der Erfindung enthält das Medium Insulin in Mengen von 0,1 bis 20 mg/l und die wasserlösliche Eisenverbindung in einer Konzentration von $10^{-5}$ bis $10^{-2}$ mol/l. Als wasserlösliche Eisenverbindung werden insbesondere Eisen(III)-citrat, Eisen(III)-sulfat, Eisen(III)-chlorid und/oder Kaliumhexacyanoferrat(III) eingesetzt.

Für eine Erleichterung bei der Kultivierung ist es im übrigen bevorzugt, dem Medium zusätzlich einen pH-Indikator zuzusetzen. Derartige Verbindungen sind dem Fachmann bekannt. Beispielhaft kann hier Phenolrot genannt werden.

Ein besonders bevorzugtes erfindungsgemäßes serumfreies Medium enthält Aminosäuren in einer Konzentration von 3 bis 700 mg/l, Vitamine von 0,001 bis 50 mg/l, Monosaccharide von 0,3 bis 10 g/l, anorganische Salze von 0,1 bis 10 000 mg/l, Spurenelemente von 0,001 bis 0,1 mg/l, Nukleoside von 0,1 bis 50 mg/l, Fettsäuren von 0,001 bis 10 mg/l, Biotin von 0,01 bis 1 mg/l, Hydrocortison von 0,1 bis 20 $\mu$g/l, Insulin von 0,1 bis 20 mg/l, Vitamin B12 von 0,1 bis 10 mg/l, Putrescin von 0,01 bis 1 mg/l, Natriumpyruvat von 10 bis 500 mg/l die wasserlösliche Eisenverbindung,sowie gegebenenfalls einen pH-Indikator und Antibiotika, wobei die vorgenannten Verbindungen gelöst in Wasser vorliegen. Die Konzentrationsangaben beziehen sich dabei jeweils auf die einzelne Komponente und nicht auf die Summe der genannten Verbindungen. So können erfindungsgemäß beispielsweise Mischungen von DMEM- und F12-Medium eingesetzt werden. Auch andere käuflich erhältliche Medien, die den Zusammensetzungen, wie sie oben angegeben sind, entsprechen, können als Grundmedien verwendet werden und dann mit den speziellen Komponenten rekombinantes Insulin und wasserlöslicher Eisenverbindung entsprechend ergänzt werden.

Im Rahmen der Erfindung ist es weiterhin besonders bevorzugt, daß das Medium zusätzlich Polyvinylalkohol und/oder Methylcellulose enthält. Die Verwendung von Polyvinylalkohol ist beispielsweise in der europäischen Patentanmeldung 0 248 656, ebenso wie von B.D. Bavister in The Journal of Experimental Zoology 217 (1981), 45-51 und Shintani et al. in Appl.Microbiol.Biotechnol. 27 (1988), 533-537 beschrieben. Auch für das erfindungsgemäße Medium ist der Zusatz von Polyvinylalkohol und/oder Methylcellulose vorteilhaft. Vorzugsweise werden die Verbindungen dabei in einer Konzentration von 0,1 bis 20 g/l eingesetzt.

Das erfindungsgemäße Medium ist insbesondere dadurch gekennzeichnet, daß es sich besonders zur Kultivierung von CHO-Zellen eignet. Insbesondere bei der Kultivierung von CHO-Zellen, die ein Fremdgen enthalten und exprimieren, hat sich die besonders gute Eignung des erfindungsgemäßen Mediums gezeigt. Im Rahmen der Erfindung handelt es sich bei dem zu exprimierenden Fremdgen in den CHO-Zellen vorzugsweise um das Gen für Erythropoietin.

Ein weiterer Gegenstand der Erfindung ist daher ein Verfahren zur Kultivierung von Säugerzellen, insbesondere CHO-Zellen in einem erfindungsgemäßen Medium. Dabei hat sich als bevorzugt eine Verfahrensweise herausgestellt, bei der man ein an bestimmten Substanzen verarmtes erfindungsgemäßes Kultivierungsmedium verwendet und dann während der Kultivierung zweimal, vorzugsweise nach 48 und 96 Stunden, jeweils 2 Vol.-% einer Mischung aus 0,1 bis 1 g/l rekombinantem Insulin, 40 bis 200 g/l Glucose und je 0,4 bis 3 g/l Asparaginsäure, Asparagin x $H_2O$, Histidin, Methionin, Prolin, Serin, 1 bis 5 g/l Cystein x HCl x $H_2O$, 1 bis 8 g/l Glutamin und 0,2 bis 2 g/l Tryptophan zusetzt, wonach die entsprechenden Konzentrationen des erfindungsgemäßen Mediums erreicht sind. Bei dieser bevorzugten Verfahrensführung hat sich gezeigt, daß das Wachstum über einen langen Zeitraum bei einer relativ hohen Rate gehalten werden kann und damit Zellmengen erhalten werden können, die höher sind als bei sofortiger Verwendung des Kultivierungsmediums und dem von Anfang an einsetzenden Verbrauch der entsprechenden Verbindungen.

Das erfindungsgemäße Verfahren wird wiederum vorzugsweise zur Kultivierung von CHO-Zellen angewandt, insbesondere von CHO-Zellen, die Erythropoietin sezernieren.

Ein weiterer Gegenstand der Erfindung ist daher ein Verfahren zur gentechnologischen Herstellung von Erythropoietin, bei dem man das Gen für Erythropoietin enthaltende CHO-Zellen in einem erfindungsgemäßen Medium bzw. gemäß einem der erfindungsgemäßen Verfahren kultiviert und das Erythropoietin aus dem Kulturmedium isoliert.

Die folgenden Beispiele sollen in Verbindung mit den Figuren die Erfindung weiter erläutern.

Fig. 1    zeigt hierbei die Lebendzelldichte in Abhängigkeit von der Kultivierungsdauer bei Verwendung verschiedener Medien und

Fig. 2    zeigt die Gesamtzelldichte in Abhängigkeit von der Kultivierungsdauer.


Beispiele


Beschreibung des in den Beispielen verwendeten Mediums


Das Medium der unten aufgeführten Beispiele enthält Aminosäuren in einer Konzentration von 3 bis 700 mg/l, Vitamine von 0,001 bis 50 mg/l, Monosaccharide von 0,3 bis 10 g/l, anorganische Salze von 0,1 bis 10000 mg/l, Spurenelemente von 0,001 bis 0,1 mg/l, Nukleoside von 0,1 bis 50 mg/l, Fettsäuren von 0,01 bis 10 mg/l, außerdem Na-Pyruvat (10 - 500 mg/l) und einen pH-Indikator. Das Medium wurde auf Basis einer aus gleichen Volumenanteilen bestehenden Mischung der Medien DMEM und F12 (Dulbeccos Modified Eagles Medium und Nutrient Mixture Hams F-12) hergestellt. An besonderen Komponenten sind enthalten:

| Biotin | 0,2036 mg/l |
|---|---|
| Hydrocortison | 3,6 µg/l |
| Insulin (rekombinant) | 5,0 mg/l |
| Putrescin | 0,1 mg/l |
| Vitamin B12 | 0,78 mg/l |


Im Medium von Beispiel 1 ist außerdem enthalten:

| Transferrin (human) | 5 mg/l |
|---|---|
| Na-Selenit | 5 $\mu$g/l |
| Polyvinylalkohol | 1 g/l |

Im Medium von Beispiel 2 ist außerdem enthalten:

| Eisen-Citrat | 124 mg/l |
|---|---|
| Polyvinylalkohol | 1 g/l |

Im Medium von Beispiel 3 ist außerdem enthalten:

| Eisen-Citrat | 124 mg/l |
|---|---|
| Methyl-Zellulose | 0,5 g/l |

In allen drei Beispielen wurden CHO-Zellen unter den üblichen Bedingungen (37°C, 5% $CO_2$) kultiviert.

Beispiel 1

Beim Einsatz eines Mediums, das 5 mg/l Transferrin, 5 $\mu$g/l Na-Selenit und 1 g/l Polyvinylalkohol enthält, wurden als maximale Werte für die Lebendzelldichte $8,1 \times 10^5$/ml (nach 189 Stunden Kulturdauer) und für die Gesamtzelldichte $10,8 \times 10^5$/ml (nach 197 Stunden Kulturdauer) erreicht.

Beispiel 2

Beim Einsatz eines Mediums, das 124 mg/l Eisen-Citrat und 1 g/l Polyvinylalkohol enthält, wurden als maximale Werte für die Lebendzelldichte $15,3 \times 10^5$/mg (nach 164 Stunden Kulturdauer) und für die Gesamtzelldichte $25,7 \times 10^5$/ml (nach 164 Stunden Kulturdauer erreicht.

Beispiel 3

Beim Einsatz eines Mediums, das 124 mg/l Eisen-Citrat und 0,5 g/l Methyl-Zellulose enthält, wurden als maximale Werte für die Lebendzelldichte $14,4 \times 10^5$/ml (nach 185 Stunden Kulturdauer) und für die Gesamtzelldichte $26,0 \times 10^5$/ml (nach 193 Stunden Kulturdauer) ereicht.

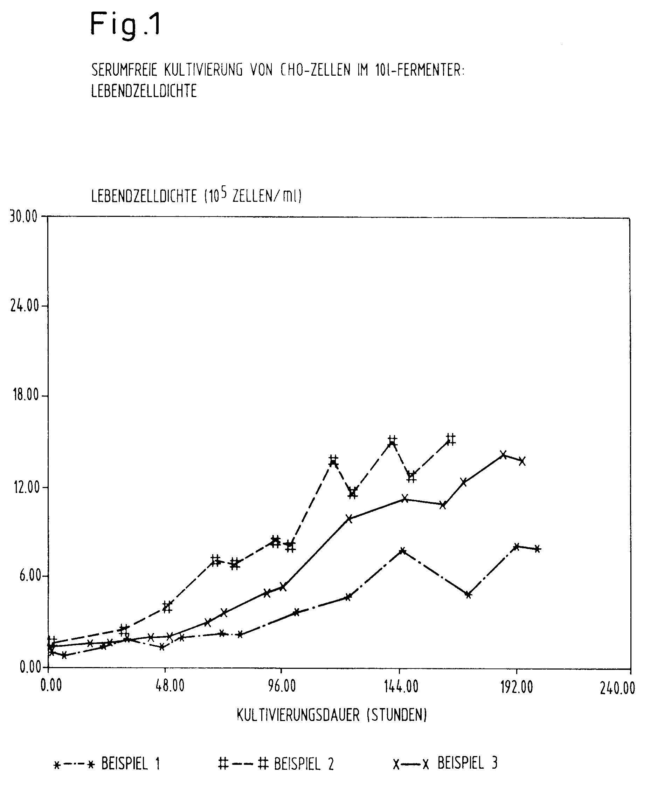

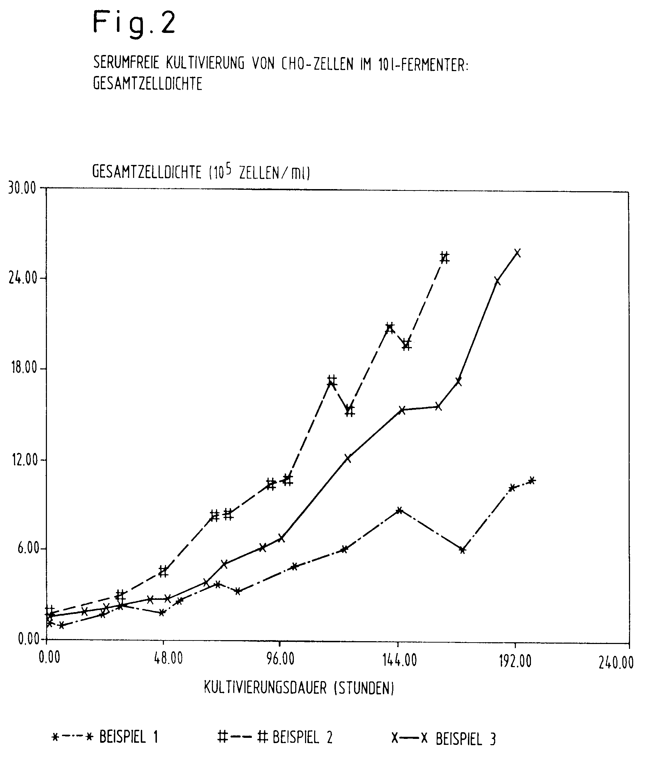

Die erhaltenen Werte für die Lebend- bzw. Gesamtzelldichte der Beispiele 1 bis 3 sind in den Figuren 1 und 2 graphisch dargestellt.

**Patentansprüche**

1. Serumfreies Medium zur Kultivierung von Säugerzellen ohne Proteinmaterial tierischen Ursprungs, enthaltend neben üblichen Inhaltsstoffen anstelle von tierischem Insulin und Transferrin rekombinantes Insulin aus Prokaryonten und eine wasserlösliche Eisenverbindung.

2. Medium nach Anspruch 1,
   **dadurch gekennzeichnet,**
   daß es Insulin in Mengen von 0,1 bis 20 mg/l und die wasserlösliche Eisenverbindung in einer Konzentration von $10^{-5}$ bis $10^{-2}$ mol/l enthält.

3. Medium nach Anspruch 1 oder 2,
   **dadurch gekennzeichnet,**
   daß es als wasserlösliche Eisenverbindung Eisencitrat, Eisensulfat, Eisenchlorid und/oder Kaliumhexacyanoferrat enthält.

**4.** Medium nach einem der vorhergehenden Ansprüche,
**dadurch gekennzeichnet,**
daß es zusätzlich einen pH-Indikator enthält.

**5.** Serumfreies Medium nach einem der vorhergehenden Ansprüche,
**dadurch gekennzeichnet,**
daß es aus Aminosäuren in einer Konzentration von 3 bis 700 mg/l, Vitaminen von 0,001 bis 50 mg/l, Monosacchariden von 0,3 bis 10 g/l, anorganischen Salzen von 0,1 bis 10 000 mg/l, Spurenelementen von 0,001 bis 0,1 mg/l, Nukleosiden von 0,1 bis 50 mg/l, Fettsäuren von 0,001 bis 10 mg/l, Biotin von 0,01 bis 1 mg/l, Hydrocortison von 0,1 bis 20 $\mu$g/l, Insulin von 0,1 bis 20 mg/l, Vitamin B12 von 0,1 bis 10 mg/l, Putrescin von 0,01 bis 1 mg/l, Natriumpyruvat von 10 bis 500 mg/l, der wasserlöslichen Eisenverbindung, sowie gegebenenfalls einem pH-Indikator und Antibiotika, gelöst in Wasser, besteht.

**6.** Medium nach einem der vorhergehenden Ansprüche,
**dadurch gekennzeichnet,**
daß es zusätzlich Polyvinylalkohol und/oder Methylcellulose vorzugsweise in einer Konzentration von 0,1 bis 20 g/l enthält.

**7.** Medium nach einem der vorhergehenden Ansprüche,
**dadurch gekennzeichnet,**
daß es sich besonders zur Kultivierung von CHO-Zellen, insbesondere von CHO-Zellen, die ein Fremdgen enthalten und exprimieren, eignet.

**8.** Medium nach Anspruch 7,
**dadurch gekennzeichnet,**
daß es sich bei dem zu exprimierenden Fremdgen um das Gen für Erythropoietin handelt.

**9.** Verfahren zur Kultivierung von Säugerzellen, insbesondere CHO-Zellen,
**dadurch gekennzeichnet,**
daß man sie in einem Medium nach einem der Ansprüche 1 bis 8 kultiviert.

**10.** Verfahren nach Anspruch 9,
**dadurch gekennzeichnet,**
daß man ein verarmtes Kultivierungsmedium verwendet und während der Kultivierung zweimal, bevorzugt nach 48 und 96 Stunden, jeweils 2 Vol.-% einer Mischung aus 0,1 bis 1 g/l rekombinantem Insulin, 40 bis 200 g/l Glucose und je 0,4 bis 3 g/l Asparaginsäure, Asparagin x $H_2O$, Histidin, Methionin, Prolin, Serin, 1 bis 5 g/l Cystein x HCl x $H_2O$, 1 bis 8 g/l Glutamin und 0,2 bis 2 g/l Tryptophan zusetzt, wonach die entsprechenden Konzentrationen eines Mediums nach einem der Ansprüche 1 bis 8 erreicht sind.

**11.** Verfahren nach Anspruch 9 oder 10,
**dadurch gekennzeichnet,**
daß man CHO-Zellen kultiviert, die Erythropoietin sezernieren.

**12.** Verfahren zur gentechnologischen Herstellung von Erythropoietin,
**dadurch gekennzeichnet,**
daß man das Gen für Erythropoietin enthaltende CHO-Zellen in einem Medium nach einem der Ansprüche 1 bis 8 bzw. gemäß einem Verfahren nach einem der Ansprüche 9 bis 11 kultiviert und das Erythropoietin aus dem Kulturmedium isoliert.

# Fig.1

SERUMFREIE KULTIVIERUNG VON CHO-ZELLEN IM 10l-FERMENTER:
LEBENDZELLDICHTE

LEBENDZELLDICHTE (10$^5$ ZELLEN/ml)

KULTIVIERUNGSDAUER (STUNDEN)

$*$—··—$*$ BEISPIEL 1     #——# BEISPIEL 2     x——x BEISPIEL 3

6

# Fig.2

SERUMFREIE KULTIVIERUNG VON CHO-ZELLEN IM 10l-FERMENTER:
GESAMTZELLDICHTE

GESAMTZELLDICHTE ($10^5$ ZELLEN/ml)

KULTIVIERUNGSDAUER (STUNDEN)

*---* BEISPIEL 1       #—— # BEISPIEL 2       X—X BEISPIEL 3